Europäisches Patentamt

(19) European Patent Office

Office européen des brevets

(11) Publication number: **0 064 989**
**B1**

(12) **EUROPEAN PATENT SPECIFICATION**

(45) Date of publication of patent specification: **18.06.86**

(21) Application number: **81903028.9**

(22) Date of filing: **22.10.81**

(86) International application number:
**PCT/US81/01417**

(87) International publication number:
**WO 82/01829 10.06.82 Gazette 82/15**

(51) Int. Cl.⁴: **B 01 J 31/40, B 01 J 23/96,**
**C 07 C 51/10, C 01 G 55/00**

(54) **PROCESS FOR THE RECOVERY OF RHODIUM CATALYST.**

(30) Priority: **24.11.80 US 209798**

(43) Date of publication of application:
**24.11.82 Bulletin 82/47**

(45) Publication of the grant of the patent:
**18.06.86 Bulletin 86/25**

(84) Designated Contracting States:
**DE FR GB NL** —

(56) References cited:
**US-A-3 845 121**
**US-A-3 887 489**
**US-A-3 927 078**
**US-A-4 046 807**
**US-A-4 188 363**
**US-A-4 234 719**

(73) Proprietor: **EASTMAN KODAK COMPANY**
**343 State Street**
**Rochester New York 14650 (US)**

(72) Inventor: **HEMBRE, Robert Thomas**
**539 Rambling Road**
**Kingsport, TN 37663 (US)**
Inventor: **COOK, Steven Leroy**
**Rt. 12, East Shipley Ferry Road, Apt.6**
**Kingsport, TN 37663 (US)**

(74) Representative: **Baron, Paul Alexander Clifford**
**et al**
**Kodak Limited Patent Department Headstone**
**Drive**
**Harrow Middlesex HA1 4TY (GB)**

Courier Press, Leamington Spa, England.

## Description

This invention relates to a process for recovering rhodium catalyst from a liquid composition that comprises such catalyst and tar, and is formed during the preparation of acetic anhydride by reacting carbon monoxide gas with methyl acetate in the presence of a catalytic combination of rhodium, lithium and an iodine compound. More particularly, this invention relates to a liquid-liquid extraction process in which the liquid composition is contacted with an aqueous extracting liquid comprising hydrogen iodide and an organic extracting liquid comprising methyl iodide.

It is known that acetic anhydride, a very useful industrial chemical, can be prepared by the carbonylation of methyl acetate in the presence of rhodium-containing catalysts. For example, the use of catalytic combinations or systems comprising rhodium and an iodine compound in the preparation of acetic anhydride by reacting carbon monoxide gas with methyl acetate has been reported in patent literature such as Belgian Patent No. 819,455, granted March 3, 1975 and Japanese Patent Application 75—47922, published April 28, 1970. These publications disclose that the reaction rate can be increased if the catalytic combination contains a promotor such as lithium. In such a process, the lithium is generally added as lithium iodide or lithium acetate.

It is also known that the formation of an undesirable by-product, so-called "tar" or "soot", is virtually unavoidable in carbonylation reactions of the type described. Furthermore, this tar cannot be easily separated from the rhodium catalyst that is present in the reaction medium which makes it very difficult to achieve efficient catalyst recovery and recycle. Due to the high cost of rhodium, efficient catalyst recovery and recycling procedures are an economic necessity in such carbonylation processes.

In processes for preparing acetic anhydride by the carbonylation of methyl acetate as previously described the catalytically active form of rhodium has been identified as the anion $[Rh(CO)_2I_2]^\ominus$. This fact, coupled with the knowledge that the tar formed in the process is a very viscous material largely hydrocarbon in nature, suggested that liquid-liquid extraction with water as an aqueous extracting liquid would be an effective technique for the separation of rhodium catalyst from the tar. Unfortunately, while water extracts much of the rhodium catalyst present in the tar, the resulting aqueous solution containing the rhodium is not stable at the elevated temperatures that are normally used in the carbonylation process. As a result of this instability, costly rhodium is lost by "plating-out" or depositing on equipment used in the process. Furthermore, if the aqueous solution containing rhodium catalyst is recycled to the carbonylation process, the amount of water present in the solution must be minimized since the presence of water leads to the formation of acetic acid which deleteriously affects the yield of acetic anhydride. Minimizing the amount of water in the solution fosters the aforementioned instability and plating out of rhodium.

It has been discovered that the inclusion of hydrogen iodide in water which is used to extract rhodium catalyst (sometimes called "rhodium catalyst values") from the tar previously described, stabilizes the catalytically active rhodium species, thereby increasing the overall efficiency of the extraction and recycling of rhodium to the carbonylation process. The aqueous hydrogen iodide extracting liquid also recovers lithium and iodine, primarily as lithium iodide. It is also essential that a second extracting liquid comprising a water-immiscible, organic solvent for the tar be used in combination with the aqueous hydrogen iodide extracting liquid. Methyl iodide is used as the organic solvent in the second extracting liquid to practice this invention because it can be recycled to the carbonylation process.

In accordance with the discovery discussed in the preceding paragraph, this invention provides a process for recovering rhodium catalyst from a liquid composition, comprising rhodium catalyst and tar, formed as a by-product during a process of preparing acetic anhydride by reacting carbon monoxide gas with methyl acetate in the presence of a catalytic combination of rhodium, lithium and an iodine compound, characterized in that the liquid composition is contacted with a first extracting liquid comprising aqueous hydrogen iodide to thereby separate rhodium catalyst in aqueous phase from the composition and a second extracting liquid comprising methyl iodide to thereby separate tar in organic phase from the composition.

In practicing the process of this invention, the liquid composition comprising rhodium catalyst and tar can be contacted with the extracting liquids simultaneously or in sequence. For example, this composition can be initially contacted with the aforementioned second extracting liquid and subsequently contacted with the aforementioned first extracting liquid.

The liquid composition comprising rhodium catalyst and tar can be removed continuously or intermittently from the carbonylation process in the form of a solution or suspension in a mixture of the compounds present in the process. This composition is normally a solution and can be removed either from the carbonylation zone or from an appropriate point in a catalyst recycle sequence. The solution can be immediately subjected to liquid-liquid extraction or it can first be concentrated by removing some of the liquids present. In commercial production facilities when rhodium is recycled to the carbonylation reaction zone, the recycle stream (which also contains some tar) is normally concentrated to some extent in the acetic anhydride recovery section of the facilities.

As previously indicated, the tar present with the rhodium catalyst in the liquid composition forms during the preparation of acetic anhydride by the carbonylation of methyl acetate. An example of such a process is the preparation of acetic anhydride by the liquid phase carbonylation of methyl acetate in the presence of a catalytic combination of rhodium, lithium and methyl iodide at elevated pressure and temperature wherein a feed containing methyl acetate is continuously introduced into a carbonylation reactor where it is reacted with carbon monoxide and a reaction mixture containing acetic anhydride is continuously removed from the reactor. Optionally, up to about 7 volume percent of hydrogen based on carbon monoxide and hydrogen gases, can also be introduced into the carbonylation reactor. In the practice of the process, the feed to the reactor is such as to maintain within the reaction mixture (1) 250 to 1300 ppm*,preferably 500 to 1000 ppm, rhodium, (2) 175 to 5000 ppm, preferably 1500 to 3700 ppm, lithium and (3) 7 to 35 weight percent metnyl iodide. The remainder of the reactor contents is mostly methyl acetate reactant and acetic anhydride product with minor amounts of such by-products as ethylidene diacetate and acetone. The reactor feed may contain a solvent such as acetic acid, e.g. in an amount that will maintain 5 to 40 weight percent in the reaction mixture. When the product is withdrawn in liquid form, the catalyst components, i.e. the rhodium, lithium and iodide as methyl iodide, are recovered from the reactor effluent and are recycled. When necessary, fresh rhodium, as rhodium chloride, rhodium acetate or other rhodium containing compound, and lithium, as lithium hydroxide, lithium iodide, lithium acetate or other lithium-containing compound are added to the catalyst recycle. The fresh rhodium and lithium can be conveniently added as a solution in acetic acid. When the iodine needs to be supplemented it may be added to the system as iodine ($I_2$), as methyl iodide or, at least in part, as lithium iodide. When reaction product is withdrawn in vapor form, all or essentially all of the rhodium and lithium catalyst components remain in the reactor and the risk of their depletion from the process is reduced considerably.

Tar formed in the carbonylation of methyl acetate as described, is particularly troublesome since the amount increases as reaction conditions such as temperature and pressure are increased to obtain acceptable space-time-yields of acetic anhydride. Space-time yield is the yield of desired product in a unit of time per unit volume of reaction space. It is conveniently expressed in terms of gram/litre/hour(s). Available analytical data do not permit absolute identification of this tar. However, the tar exhibits certain spectral features that are very reproduceable but are very difficult to resolve. Based upon combined information of IR (Infrared), H NMR (Hydrogen Nuclear Magnetic Resonance), C NMR (Carbon Nuclear Magnetic Resonance) and elemental analysis, certain aspects of the "structure" may be proposed; but the poor resolution in the H and C NMR spectra thwart hopes for absolute identification and are suggestive of a highly amorphous material. The C NMR shows two major broad band absorptions of approximately equal intensity, one in the alkyl region (13—45 $\delta$) and the other in the aromatic region (120—140 $\delta$). For the same material, the H NMR shows almost no aromatic protons relative to the alkyl bands at .9—1.7 and 1.6—3.0 $\delta$. In combination, these two spectra, therefore, suggest a polyalkylated aromatic material substituted to the exclusion of aromatic protons. Additionally, the C shows some very minor absorptions assignable to carbonyl moieties, a feature strongly suggested by the IR band at 1700 $cm^{-1}$. The IR also suggests carbon-oxygen (1180 $cm^{-1}$) and oxygen-hydrogen (36—3300 $cm^{-1}$) bonds. The elemental analysis substantiates the expectations of aromatic. unsaturation showing .60 unsaturations per carbon and an empirical formula of $C_{36}H_{41}O_4I_{15}$. Despite some ambiguity in the structure of the tar, as a practical matter, this tar is readily soluble in methyl iodide and it has proven amenable to the liquid-liquid extraction process of this invention.

The amounts of aqueous hydrogen iodide and methyl iodide extracting liquids that can be used to satisfactorily separate the rhodium catalyst from the liquid composition containing the catalyst and tar will vary substantially depending on a number of factors such as the concentration of the tar in the liquid composition, the amount of rhodium catalyst present in the tar, the extraction efficiency desired, and the type of apparatus that is used. The catalytically active anionic rhodium species that is present in the liquid composition is soluble in the quantity of methyl iodide that is normally used in the carbonylation process, but in the presence of water this species preferentially dissolves in the aqueous phase which forms. Thus, the use of larger amounts of water ensures that more of the total rhodium catalyst will be present in the aqueous phase. Also, the use of large amounts of water permits optimum phase separation between the aqueous and organic phases by avoiding emulsion formation. However, the use of relatively large amounts of water requires the use of a concentration step in order to minimize the amount of acetic anhydride decomposition that is caused by water in the carbonylation process. The use of very large amounts of water can, therefore, require the use of a considerable amount of energy for a concentration step which increases operating costs disproportionately to the value of the rhodium catalysts recovered. In practicing this invention, the liquid composition withdrawn from the carbonylation reactor can be concentrated to a weight of at least 50 percent, preferably, at least 30 percent, to avoid the presence of undue amounts of water without the detrimental economical consequences discussed previously. Also, concentrating this liquid composition by distillation provides a distillate containing methyl iodide, methyl acetate, acetic anhydride and acetic acid which can be recycled to the carbonylation reactor.

The amount of hydrogen iodide employed in practicing this invention varies depending on several factors, including the amount of catalytic components present in the liquid composition to be extracted and the temperature to which this composition is heated. Normally, sufficient hydrogen iodide is used so that the pH of the aqueous phase is 1 or less, preferably in the range of 0.6 to 0.8. The concentration of the hydrogen iodide solution can be varied from a rather concentrated aqueous hydrogen iodide solution of 47

*ppm: parts by weight per million

3

weight percent, to a more dilute solution of 6 weight percent. The amount of methyl iodide employed in practicing this invention is at least 2 parts by weight per part, by weight, of the liquid composition subjected to extraction. Methyl iodide weight ratios of 3 to 5 (same basis) give good results.

In practicing the process of this invention, it is convenient to remove a portion of liquid compositions containing tar and catalyst components from the carbonylation process and feed this portion to a tank. The liquid composition can be removed from the process continuously or intermittently. The liquid composition then can be fed periodically to another tank fitted with means for agitation. The make-up of the liquid composition will vary depending, for example, upon the point from which it is taken from the carbonylation process. Initially, low boiling components such as methyl iodide and methyl acetate along with some acetic acid and acetic anhydride are removed to provide a concentrated solution of the tar and rhodium catalyst in acetic anhydride and acetic acid. Methyl iodide and the aqueous hydrogen iodide are added to the concentrated solution with agitation. In batch operations, best results are usually obtained when the methyl iodide is added first to a concentrated solution previously cooled to just below the boiling point of methyl iodide. After partitioning, agitation is stopped and the aqueous and organic layers are allowed to separate. The methyl iodide phase containing the tar is removed from the bottom of the tank. The remaining aqueous solution of hydrogen iodide and rhodium, lithium and iodine catalyst components in water and acetic acid is distilled under vacuum to remove most of the water, e.g. 90%. The remaining solution can then be recycled to the carbonylation process.

The methyl iodide and tar may be separated from each other by distillation and the viscous residue can be treated further to remove any iodine and rhodium present. The water and methyl iodide recovered in the process can be recycled for use in a later extraction.

The process of this invention can be carried out continuously by feeding the liquid composition obtained from the carbonylation process, aqueous hydrogen iodide and methyl iodide simultaneously to a tank where these materials are mixed, and then feeding the mixture to about the midpoint of an extracting column. An aqueous stream containing most of the rhodium catalyst is removed from the top of the column and the methyl iodide containing the tar is removed from the bottom. Normally, additional aqueous hydrogen iodide and methyl iodide are added to the lower and upper portions, respectively, of the extraction column.

The practice of the process of this invention is further illustrated by the following examples. The liquid compositions containing rhodium catalyst and tar or simply the tar employed in the liquid-liquid extraction procedures of the following examples, were obtained from a carbonylation process using a 3.5 liter reactor consisting of five feet five inches (1.65 m) of two-inch (5 cm) diameter pipe. A gas mixture of carbon monoxide and 5 volume percent hydrogen, based on carbon monoxide and hydrogen gases, was fed through a gas sparger at the bottom of the reactor. Through a reactor feed line, located above the sparger, was fed a mixture containing methyl acetate, acetic acid, acetic anhydride, methyl iodide, lithium and rhodium at an average rate of 12,600 grams/hour. The composition of the feed stream was 18—20%, by weight, methyl iodide, 55—40%, by weight, methyl acetate, 15—20%, by weight, acetic acid, 15—20%, by weight, acetic anhydride, 750 ppm rhodium and 3500 ppm lithium. Using this system, acetic anhydride was produced at 750 psig (5273 kPa) and 190°C. at a space-time yield of 600 grams/liter/hour. The reactor contents overflowed from the top of the reactor to a reactor separator where some of the unreacted carbon monoxide and other gases were separated from the liquid and purged from the system. The liquid from the separator passed through a valve which reduced the pressure from 750 psig (5273 kPa) to 10—20 psig (170—239 kPa). The liquid passed through a flash evaporator, where 80 to 90% of the material was vaporized and entered a pot (about 1 psig (108 kPa) where the vapor and liquid were separated. The liquid, which was mainly acetic acid and acetic anhydride in which the rhodium and lithium catalyst components were dissolved along with minor amounts of methyl iodide and methyl acetate, was recycled to the reactor. The vapors from the pot were fed to a column in which the temperature was maintained at 140°C. at the base and 100°C. at the top. Crude acetic anhydride suitable for further refining was removed from the lower portion of the column. The low boiling components (methyl acetate, methyl iodide and some acetic acid) were taken overhead and fed to a tank to which makeup methyl acetate was also fed. The contents of the tank were continuously fed to the reactor feed line. The liquid compositions containing rhodium catalyst and tar used in the examples were obtained from the catalyst recycle stream ("so-called catalyst heel").

Example 1

A sample of catalyst heel was taken after 112 hours of acetic anhydride production, as described in the preceding paragraph. During this time the weight ratio of tar formation to acetic anhydride production was 0.00022. A 152.2 g. portion of the catalyst heel was concentrated to about 40 g. on a rotary evaporator (95°C., 30 minutes, 5—10 torr (.67—1.33 kPa)), dissolved in methyl iodide/13 weight percent aqueous hydrogen iodide, partitioned, and separated to give 79.5 g. aqueous phase and 70.9 g. organic phase. Two additional extractions of the organic phase with the aqueous HI followed.

The rhodium analytical results and balance calculations are listed below. Analytical samples (Rh (samples)) were dissolved in 10% (v/v) HCl-Dimethylformamide and compared with previously prepared standards in the rhodium concentration ([Rh]) range of 0—20 ppm where adsorption is linearly dependent upon concentration.

Rhodium balance: $Rh_{initial} = 199.6$ mg

|  |  |  |
|---|---|---|
| Rh (aq.) final | = | 198.0 mg (99.2%) |
| Rh (org.) final | = | 0.8 mg (0.4%) |
| Rh (samples) | = | 0.2 mg (0.1%) |

$$\% \text{ Accountability} = \frac{\text{total Rh final}}{\text{total Rh}_{initial}} \times 100 = 99.7$$

$$\% \text{ Efficiency} = \frac{\text{Rh (aq.) final}}{\text{total Rh}_{initial}} \times 100 = 99.2$$

Example 2

This example illustrates that tar from which all of the volatile components have been removed may be submitted to a second extraction to recover additional rhodium.

1.95 g of dried tar, [Rh]=7938 ppm (C/Rh=739 by elemental analysis), Rh initial 15.4 mg., obtained according to the procedure of Example 1 was dissolved in 31.7 g. methyl acetate, 13.7 g. acetic acid, 12.7 g. methyl iodide, and 3.25 g. lithium iodide trihydrate. Following concentration on a rotary evaporator (90°C., 45 minutes, 5—10 torr (.67—1.33 kPa)), dissolution in methyl iodide/concentrated aqueous HI (47%), partitioning, and separation the methyl iodide was stripped from the organic phase leaving 1.90 g. dry granular solid; [Rh]=1139 ppm, (C/Rh=4645 by elemental analysis), 2.2 mg. The aqueous phase, 102.8 g., contained $[Rh]_{(aq)}$=127.0 ppm, 13.1 mg.

Rhodium balance: $Rh_{initial}$=15.4 mg Rh (aq), final=13.1 mg (85.0%)

Rh (tar), final=2.2 mg (14.3%)

Accountability=99.3%
Efficiency=85%

Example 3

The following runs illustrate the stabilizing effect of hydrogen iodide on the rhodium catalyst species in aqueous phase. Thus, a comparison of the results obtained in Run 1 (aqueous hydrogen iodide extractant) with those of Run 2 (water extractant) demonstrates that the use of hydrogen iodide increases the amount of rhodium catalyst which remains in the aqueous phase, even after reflux. Accordingly, a total of 307 ppm of Rh was recovered in Run 1 from 19.3 g. of tar while only 306.2 ppm of Rh was recovered in Run 2 from 23.5 g. of tar. Also, the comparison set forth in Run 3 between the use of aqueous hydrogen iodide extractant and water extractant clearly illustrates that the presence of the hydrogen iodide in the aqueous phase increases the amount of rhodium catalyst in that phase and improves the efficiency of rhodium recovery.

Run 1

19.3 g of tar and 199.9 g. concentrated aqueous HI (47%) were placed in a 250 ml. round bottom flask with a magnetic stir bar. After stirring 15 minutes, a sample of aqueous solution was extracted and analyzed; $[Rh]_{initial}$=151 ppm. The solution was then heated to reflux for three hours and a second sample extracted and analyzed; $[Rh]_{final}$=156 ppm.

Run 2

23.5 g. of tar and 100 ml. $H_2O$ were placed in a 250 ml. round bottom flask with a magnetic stir bar. After stirring 15 minutes a sample of aqueous solution was extracted and analyzed.

$[Rh]_{initial}$=304.7 ppm. The solution was then heated to reflux for three hours and a second sample extracted and analyzed; $[Rh]_{final}$=1.5 ppm.

Run 3

5.0 g. of tar was dissolved in 45.6 g. methyl iodide and 50 ml. of concentrated aqueous HI (47%). After partitioning and separation, a sample of the aqueous phase was placed in a vial and 10 ml. were diluted to 75 ml. Atomic adsorption analyses of undiluted ($[Rh]_{conc.}$) and dilute ($[Rh]_{dil.}$) samples were taken to provide initial rhodium content. The solutions were allowed to sit for 80 hours at room temperature, then analyzed for final rhodium content. The results are shown below:

$$[Rh]_{conc., initial} = 309 \text{ ppm}$$

$$[Rh]_{conc., final} = 229 \text{ ppm}$$

$$[Rh]_{dil., initial} = 41 \text{ ppm}$$

$$[Rh]_{dil., final} = 44 \text{ ppm}$$

A sample prepared as above with water only in place of concentrated aqueous HI was also prepared and allowed to sit for 80 hours at room temperature. The analysis of this sample ($[Rh]_{aq.}$) was as follows:

$$[Rh]_{aq., initial} = 169 \text{ ppm}$$

$$[Rh]_{aq., final} = 53 \text{ ppm}$$

Examples 4—9

Samples of catalyst heel were taken after 120, 274, 340, 438, 508 and 796 hours of acetic anhydride production as described in the paragraph preceding Example 1. Each of the samples along with acetic acid were placed in a 3 neck flask (3 liter) fitted with a stirrer, means for adding materials to the flask, and means for distilling off, condensing and collecting liquid materials from the flask. The flask also had a drain for removing liquid which could be fed to a second 3 neck flask (1 liter). This second flask had a stirrer, was fitted with a condensing means so that vaporized material could be removed, condensed and collected and had a drain tube.

The catalyst heel was concentrated in the first flask by distilling under vacuum one hour at 400 torr (53.3 kPa) initial pressure and a final pressure and temperature of 150 torr (20 kPa) and 95°C. Methyl iodide and then dilute aqueous HI were added to the cooled residue from the flask over a total time of 20 minutes. External cooling was employed to maintain the temperature between 25 and 50°C. The mixture was stirred for an additional five minutes and then the aqueous and organic layers were permitted to separate over a period of 10 minutes. The drain was opened and the methyl iodide layer containing the tar was transferred to the 1 liter flask. Most of the methyl iodide was stripped off at atmospheric pressure and a temperature of 41—50°C. While the tar residue was hot, it was drained into another container. Since the tar residue contained some methyl iodide and acetic acid, a small sample was stripped at 6 torr (0.8 kPa), 100°C. for 30 minutes to provide a more accurate value for tar content.

The aqueous HI (containing rhodium catalyst) remaining in the 3 liter flask was stripped at 140—160 torr (18.7—21.3 kPa) until the temperature reached 95—97°C. The concentrated solution then was drained from the flask, combined with the distillate first collected in the process and added to the feed line to carbonylation reactor described in the paragraph preceding Example 1.

The amounts of materials used and recovered, analytical data pertaining thereto and the rhodium extraction efficiency for each of Examples 4—9 are shown in the following Table II.

Step I of the Table shows the amounts of catalyst heel (catalyst recycle solution (CRS)) and acetic acid (HOAc) initially added, the amount and composition in weight percent, of the distillate collected (MeI=methyl iodide, MeOAc=methyl acetate, Ac₂O=acetic anhydride, EDA=ethylidene diacetate) and the amount of rhodium in the residue.

Step II of the table shows the amounts of methyl iodide and concentrated (47%) aqueous HI and water used in the extraction.

Step III shows the amount of methyl iodide recovered from the 1 liter flask, the amount of tar, both with and without volatiles, the amount of rhodium in the tar and the extraction efficiency (EE).

Step IV of the Table shows the amount of the distillate, and the amount of acetic acid contained therein, resulting from the concentration (water removal) of the aqueous phase of the aqueous/organic solvent extraction step.

# 0 064 989

### TABLE II

| | | Examples | | | | | |
|---|---|---|---|---|---|---|---|
| | | 4 | 5 | 6 | 7 | 8 | 9 |
| Step 1 Additions, g. | CRS | 580.0 | 510.2 | 562.4 | 610.0 | 620.8 | 510.3 |
| | HOAc | 131.2 | 83.0 | 77.4 | 66.7 | 103.1 | 15.7 |
| | Distillate, g. | 458.7 | 400.0 | 390.0 | 396.1 | 439.4 | 302.1 |
| | MeI | 2.0 | 1.1 | 1.7 | 1.0 | 0.9 | 1.0 |
| | MeOAc | 15.2 | 9.6 | 22.2 | 14.1 | 12.4 | 7.8 |
| | Acetone | None | 0.3 | 1.9 | 0.2 | 2.5 | Trace |
| | HOAc | 56.4 | 58.1 | 49.8 | 50.2 | 48.7 | 32.2 |
| | $Ac_2O$ | 25.9 | 30.1 | 24.0 | 34.1 | 34.9 | 51.7 |
| | EDA | 0.3 | 0.8 | 0.3 | 0.4 | 0.4 | 0.9 |
| | Rh, g. | 2.553 | 1.022 | 1.608 | 2.295 | 1.973 | 1.204 |
| Step II | MeI | 938.7 | 892.9 | 901.0 | 904.1 | 900.7 | 888.2 |
| | Conc. HI | 151.1 | 150.1 | 150.0 | 150.1 | 149.9 | 152.4 |
| | $H_2O$ | 743.3 | 758.1 | 750.0 | 744.0 | 740.7 | 750.0 |
| Step III | MeI | 878.8 | 842.0 | 805.2 | 815.8 | 818.4 | 799.5 |
| | Tar, g. | 87.7 | 45.3 | 39.7 | 47.5 | 63.0 | 63.2 |
| | Tar less volatiles | —— | 33 | 28 | 29 | 44 | 47 |
| | Rh, mg | 248 | 140 | 236 | 358 | 75 | 66 |
| | EE, % | 90.3 | 86.3 | 85.3 | 84.4 | 96.2 | 94.5 |
| Step IV Distillate, g. | | 779.2 | 835.7 | 845.1 | 807.7 | 783.4 | —— |
| | HOAc, g. | 96.6 | 154.8 | 234.6 | 295.2 | 97.8 | —— |

## Claims

1. A process for recovering rhodium catalyst from a liquid composition, comprising rhodium catalyst and tar, formed as a by-product during a process of preparing acetic anhydride by reacting carbon monoxide gas with methyl acetate in the presence of a catalytic combination of rhodium, lithium and an iodine compound,

characterized in that said liquid composition is contacted with a first extracting liquid comprising aqueous hydrogen iodide to thereby separate rhodium catalyst in aqueous phase from said composition and a second extracting liquid comprising methyl iodide to thereby separate tar in organic phase from said composition.

2. A process according to claim 1, characterized in that sufficient hydrogen iodide is used such that said aqueous phase has a pH of 1 or less.

3. A process according to claim 1 or 2, characterized in that said liquid composition is substantially simultaneously contacted with said extracting liquids.

7

## 0 064 989

4. A process according to claim 1 or 2, characterized in that said liquid composition is initially contacted with said second extracting liquid.

**Patentansprüche**

1. Verfahren zur Wiedergewinnung eines Rhodiumkatalysators aus einem flüssigen, den Rhodiumkatalysator und Teer enthaltenden Produktes, das als Nebenprodukt bei der Herstellung von Essigsäureanhydrid durch Umsetzung von Kohlenmonoxidgas mit Methylacetat in Gegenwart einer katalytischen Kombination aus Rhodium, Lithium und einer Iodverbindung gebildet wird,

dadurch gekennzeichnet, daß das flüssige Produkt mit einer ersten Extraktionsflüssigkeit mit wäßrigem Iodwasserstoff zur Abtrennung von Rhodiumkatalysator in wäßriger Phase von dem Produkt und mit einer zweiten Extraktionsflüssigkeit mit Methyliodid zur Abtrennung von Teer in organischer Phase von dem Produkt in Kontakt gebracht wird.

2. Verfahren nach Anspruch 1, dadurch gekennzeichnet, daß ausreichend Iodwasserstoff verwendet wird, derart, daß die wäßrige Phase einen pH-Wert von 1 oder weniger hat.

3. Verfahren nach Anspruch 1 oder 2, dadurch gekennzeichnet, daß das flüssige Produkt praktisch gleichzeitig mit den zur Extraktion verwendeten Flüssigkeiten in Kontakt gebracht wird.

4. Verfahren nach Anspruch 1 oder 2, dadurch gekennzeichnet, daß das flüssige Produkt zunächst mit der zweiten Extraktionsflüssigkeit in Kontakt gebracht wird.

**Revendications**

1. Procédé pour la récupération d'un catalyseur au rhodium à partir d'une composition liquide, comprenant du catalyseur au rhodium et du goudron, formée ent tant que sous-produit dans un procédé de préparation d'anhydride acétique par réaction de monoxyde de carbone avec de l'acétate de méthyle en présence d'une combinaison catalytique de rhodium, de lithium et d'un composé d'iode, caractérisé en ce que l'on met cette composition liquide en contact avec un premier liquide d'extraction comprenant de l'iodure d'hydrogène aqueux de façon à séparer ainsi le catalyseur au rhodium en phase aqueuse de cette composition et avec un second liquide d'extraction comprenant de l'iodure de méthyle de façon a séparer ainsi le goudron en phase organique de la dite composition.

2. Procédé selon la revendication 1, caractérisé en ce que l'on utilise suffisamment d'iodure d'hydrogène pour amener le pH de la phase aqueuse à une valeur égale ou inférieure à 1.

3. Procédé selon la revendication 1 ou 2, caractérisé en ce que l'on met la composition liquide en contact à peu près simultanément avec les dits liquides d'extraction.

4. Procédé selon la revendication 1 ou 2, caractérisé en ce que l'on met initialement la composition liquide en contact avec le dit second liquide d'extraction.

8